# EUROPÄISCHE PATENTSCHRIFT

(11) **EP 2 451 535 B1**
(45) Veröffentlichungstag und Bekanntmachung des Hinweises auf die Patenterteilung: **11.09.2019**
(21) Anmeldenummer: 10730727.4
(22) Anmeldetag: 06.07.2010
(51) Int. Cl.: A61Q 17/04, A61K 8/40, A61K 8/63

(54) **GLYCYRRHETINSÄURE ENTHALTENDE KOSMETISCHE ZUBEREITUNG**
COSMETIC COMPOSITION CONTAINING GLYCYRRHETINIC ACID
COMPOSITION COSMÈTIQUE CONTENANT DE L'ACIDE GLYCYRRHÉTINIQUE

(30) Priorität: 06.07.2009 DE 102009032245
(43) Veröffentlichungstag der Anmeldung: 16.05.2012
(73) Patentinhaber: Beiersdorf AG, 20253 Hamburg (DE)
(72) Erfinder: ZANFORLIN TREDE, Lucia, 22769 Hamburg (DE); STEIKERT, Claudia, 22529 Hamburg (DE); BLOHM, Alexandra, 20257 Hamburg (DE)
(86) Internationale Anmeldenummer: PCT/EP2010/004127
(87) Internationale Veröffentlichungsnummer: WO 2011/003597

(56) Entgegenhaltungen:
- WO-A2-2004/009038
- DE-A1-102007 041 473
- US-A1- 2004 047 818
- TSAI T H ET AL: "Determination and UV spectral identification of 18alpha-glycyrrhetinic acid and 18beta-glycyrrhetinic acid for stability studies", INTERNATIONAL JOURNAL OF PHARMACEUTICS, ELSEVIER BV, NL, Bd. 84, Nr. 3, 20. August 1992 (1992-08-20), Seiten 279-281, XP025557721, ISSN: 0378-5173, DOI: 10.1016/0378-5173(92)90166-Y [gefunden am 1992-08-20]

## Beschreibung

Die vorliegende Erfindung betrifft eine kosmetische Zubereitung enthaltend Glycyrrhetinsäure und Octocrylen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. Ein Hauptnachteil UV-Filter enthaltender kosmetischer Zubereitungen ist dabei ihre Stabilität. Dabei unterscheidet der Fachmann einerseits zwischen der Photostabilität der Zubereitungen, d.h. ihrer Stabilität gegenüber dem Sonnenlicht und der Lagerstabilität der Zubereitungen, worunter der Fachmann in erster Linie die mikrobielle Stabilität und die Temperaturstabilität versteht. Beide Effekte gilt es dabei sorgfältig zu unterscheiden. So kennt der Fachmann beispielsweise UV-Filter wie 2-Ethylhexyl-2-cyano-3,3-diphenylacrylat (Octocrylen), die äußerst photostabil sind und teilweise sogar zur Photostabilisierung anderer UV-Filter verwendet werden, die andererseits aber nur begrenzt lagerstabil sind und durch länger andauernde thermische Belastung zerstört werden.

Die mangelnde Stabilität von Sonnenschutzmitteln hat zu der Expertenempfehlung geführt, derartige Produkte nicht länger als eine Saison zu verwenden und anschließend durch neue Zusammensetzungen zu ersetzen. Dadurch landen jedes Jahr große Mengen an "angebrochenen" Sonnenschutzprodukten im Hausmüll, was eine nicht unerhebliche Ressourcenverschwendung darstellt.

Es war daher die Aufgabe der vorliegenden Erfindung die Mängel des Standes der Technik zu beseitigen und Octocrylen-haltige kosmetische Zubereitungen zu entwickeln, die eine höhere Stabilität, insbesondere Temperaturstabilität aufweisen.

Gelöst wird die Aufgabe durch eine kosmetische Zubereitung enthaltend
a) Glycyrrhetinsäure,
b) 2-Ethylhexyl-2-cyano-3,3-diphenylacrylat (Octocrylen) und
c) einen oder mehrere Parfümstoffe, dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 2-Phenylbenzimidazol-5-sulfonsäuresalze und/oder Titandioxid als weitere UV-Filter enthält.

Gelöst wird die Aufgabe ferner durch die Verwendung von Glycyrrhetinsäure zur Stabilisierung von 2-Ethylhexyl-2-cyano-3,3-diphenylacrylat (Octocrylen) in kosmetischen Zubereitungen.

Insbesondere wird die Aufgabe gelöst durch die Verwendung von Glycyrrhetinsäure zur Erhöhung der Temperaturstabilität von 2-Ethylhexyl-2-cyano-3,3-diphenylacrylat (Octocrylen) haltigen kosmetischen Zubereitungen.

Zwar kennt der Fachmann die DE 10 2006 009783, DE 10 2006 009 850 und DE 10 2007 041 473, doch konnten diese Schriften nicht den Weg zur vorliegenden Erfindung weisen, da sich diese Schriften mit der Haut bräunenden Wirkung von Glycyrrhetinsäure befassen und keinerlei Hinweise auf die Stabilitätserhöhung bieten. Auch die WO 2004/009038, die sich mit den antibakterielllen Eigenschaften der Glycyrrhetinsäure in kosmetischen Zubereitungen beschäftigt, konnte nicht den Weg zur vorliegenden Erfindung weisen. Ferner kennt der Fachmann die WO 2004/009038, US 2004/0047818 und Tsai Th. Et al.: "Determination ans UV spectral identification of 18alpha-glycyrrhetinic acid ans 18beta-glycyrrhetinic acid for stability studies" Int. J. of Pharmaceutics, Elsevier BV, Bd. 84, Nr. 3, 20.August 1992, Seiten 279-281 XP0255557721, sowie den Datenbank-Eintrag in der gnpd-Datenbank "Mintel", Record ID 920290 "Sea & Tropics Tanning Body", die ebenfalls nicht den Weg zur vorliegenden Erfindung weisen konnten.

Die Begriffe "erfindungsgemäß", "erfindungsgemäß vorteilhaft" etc. beziehen sich im Rahmen der vorliegenden Offenbarung sowohl auf die erfindungsgemäße Zubereitung als auch auf die erfindungsgemäße Verwendung, auch wenn dies nicht besonders vermerkt ist. Ausführungen zur erfindungsgemäßen Zubereitung schließen auch die erfindungsgemäße Verwendung mit ein. Die erfindungsgemäß vorteilhaften Ausführungsformen können dazu dienen, den erfindungsgemäßen Effekt weiter zu optimieren.

Es ist erfindungsgemäß vorteilhaft, wenn die erfindungsgemäße Zubereitung Glycyrrhetinsäure in einer Konzentration von 0,01 bis 1 Gew,-%, bezogen auf das Gesamtgewicht der Zubereitung, enthält.

Ferner sind die erfindungsgemäß vorteilhaften Ausführungsformen der vorliegenden Erfindung dadurch gekennzeichnet, dass die Zubereitung 2-Ethylhexyl-2-cyano-3,3-diphenyl-acrylat (Octocrylen) in einer Konzentration von 0,5 bis 7,5 Gew.-%, bezogen auf das Gesamtgewicht der Zubereitung, enthält.

Es ist dabei erfindungsgemäß von Vorteil, wenn in der erfindungsgemäßen Zubereitung das Gewichtsverhältnis von Glycyrrhetinsäure zu 2-Ethylhexyl-2-cyano-3,3-diphenylacrylat (Octocrylen) von 0,02 bis 0,15 beträgt.

Die erfindungsgemäße Glycyrrhetinsäure ist der Fachwelt auch bekannt unter dem chemischen Namen 3 β-Hydroxy-11-oxoolean-12-en-30-säure (Freinamen: Enoxolon, Bioson).

Es ist erfindungsgemäß vorteilhaft, wenn die erfindungsgemäße Zubereitung einen oder mehrere weitere UV-Filter gewählt aus der Gruppe der Verbindungen Phenylen-1,4-bis-(2-benzimidae)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexyl-salicylat; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäureamylester; 4-Methoxybenzalmalonsäuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat;; Dimethicodiethylbenzalmalonat; 3-(4-(2,2-bis Ethoxycarbonylvinyl)phenoxy)propenyl)-meth-oxysiloxan/Dimethylsiloxan - Copolymer; Dioctylbutylamidotriazon (INCI: Diethylhexyl-Butamidotriazone); 24-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate enthält.

Die erfindungsgemäß vorteilhaften Merocyanine werden dabei gewählt aus der Gruppe der Verbindungen

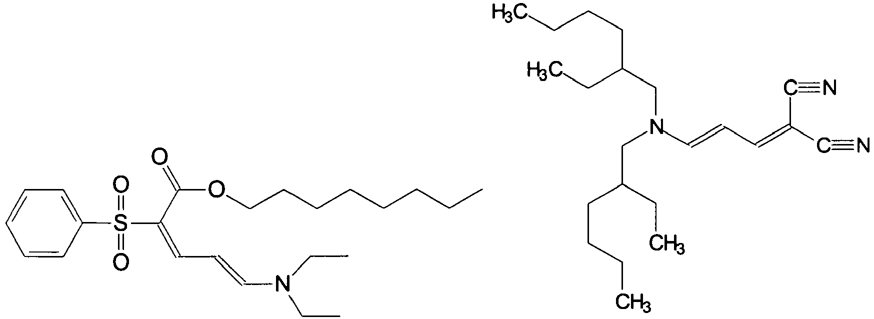

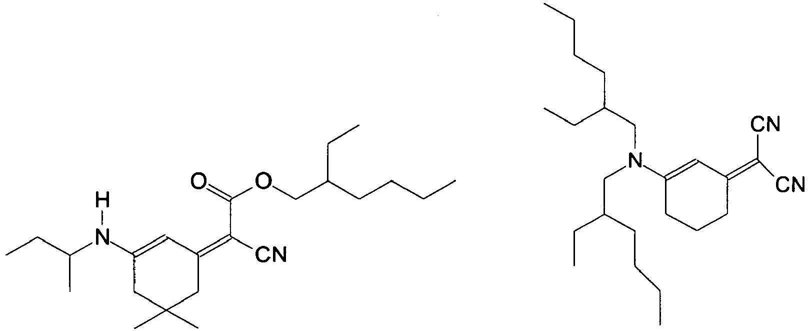

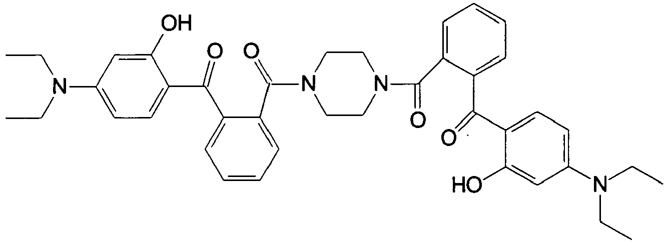

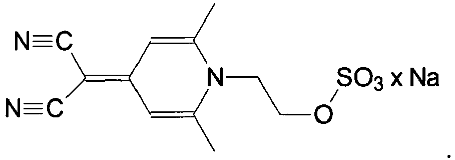

Als erfindungsgemäß vorteilhaftes Piperazinderivat kann dabei die folgende Verbindung eingesetzt werden:

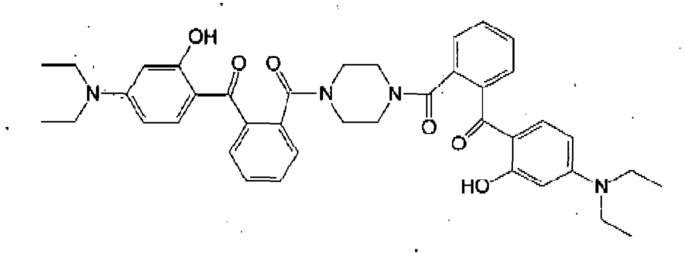

Es ist erfindungsgemäß vorteilhaft, wenn die erfindungsgemäße.Zubereitung frei ist von p-Methylbenzylidencampher.

Es ist erfindungsgemäß bevorzugt, wenn die erfindungsgemäße Zubereitung 4-(tert-Butyl 4'-methoxydibenzoylmethan als weiteren UV-Filter enthält. In einem solchen Falle ist es erfindungsgemäß vorteilhaft, wenn die Zubereitung 4-(tert.-Butyl)-4'-methoxydibenzoylmethan in einer Konzentration von 0,5 bis 5 Gew.-%, bezogen auf das Gesamtgewicht der Zubereitung, enthält.

Ebenso ist es erfindungsgemäß bevorzugt, wenn die erfindungsgemäße Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 2-Phenylbenzimidazol-5-sulfonsäuresalze und/oder Titandioxid als weitere UV-Filter enthält.

Erfindungsgemäß besonders bevorzugt sind Zubereitungen, die sowohl 4-(tert.-Butyl)-4'-methoxydibenzoylmethan als auch 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 2-Phenylbenzimidazol-5-sulfonsäuresalze und/oder Titandioxid als weitere UV-Filter enthalten.

Erfindungsgemäß ganz besonders bevorzugt sind Zubereitungen, die als UV-Filtersystem eine Mischung aus 2-Ethylhexyl-2-cyano-3,3-diphenylacrylat (Octocrylen), 4-(tert.-Butyl)-4'-methoxydibenzoylmethan, 2.4-Bis-{[4-(2-ethyl-hexyloxy)-2-hydroxyl-phenyl}-6-(4-methoxyphenyl)-1,3,5-triazin (INCI: Bis-Ethylhexyloxyphenol Methoxyphenyl Triazin), 2-Phanylbenzimidazol-5-sulfonsäuresalze und Titandioxid enthalten.

Es ist erfindungsgemäß, wenn die erfindungsgemäße Zubereitung einen oder mehrere Parfümstoffe enthält.

Erfindungsgemäß bevorzugte Ausführungsformen im Sinne der vorliegenden Erfindung sind dadurch gekennzeichnet, dass die Zubereitung einen oder mehrere Parfümstoffe gewählt aus der Gruppe der Verbindungen Linalool, Benzyl Benzoate, Hydroxyisohexyl 3-Cyclohexene Carboxaldehyd, Hexyl Cinnamal, Benzyl Salicylate, Citronellol, Coumarin, enthält.

Es ist erfindungsgemäß vorteilhaft, wenn die Gesamtkonzentration an Parfümstoffen in der Zubereitung von 0,00001 bis 1 Gew.-%, bezogen auf das Gesamtgewicht der Zubereitung, beträgt.

Die erfindungsgemäße Zubereitung kann als wässrige oder wässrig-alkoholische Zubereitung oder Gel, als Hydrodispersion oder als Emulsion vorliegen. Die erfindungsgemäße bevorzugte Ausführungsform ist dabei die Emulsion.

Liegt die erfindungsgemäße Zubereitung in Form einer Emulsion vor, dann ist es erfindungsgemäß bevorzugt, wenn die Emulsion eine O/W-Emulsion (Öl-in-Wasser-Emulsion) darstellt.

Liegt die erfindungsgemäße Zubereitung in Form einer O/W-Emulsion vor, so ist sie erfindungsgemäß bevorzugt dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat, Sucrose Polystearat, Natrium Stearoyl Glutamat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Außerdem ist es erfindungsgemäß vorteilhaft Kaliumcetylphosphat als Emulgator einzusetzen.

Erfindungsgemäß besonders bevorzugt ist dabei, wenn die Zubereitung mit Hilfe der Kombination aus Cetearylalkohol, PEG-40 Rizinusöl, Natriumcetearylsulfat und Glycerylstearat zu emulgieren.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein. Erfindungsgemäß vorteilhafte Ausführungsformen der vorliegenden Erfindung sind dadurch gekennzeichnet, dass die erfindungsgemäße Zubereitung ein oder mehrere Verbindungen gewählt aus der Gruppe der Alkohole, Diole, Glycole, Glycerin enthält.

Es ist erfindungsgemäß vorteilhaft, wenn die erfindungsgemäße Zubereitung Feuchthaltemittel wie Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure, Hyaluronsäure, Chitosan und/oder Harnstoff enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl-oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner.

Erfindungsgemäß vorteilhafte Ausführungsformen der vorliegenden Erfindung sind dadurch gekennzeichnet, dass die Zubereitung als Verdickungsmittel eine oder mehrere Verbindungen gewählt aus der Gruppe der Verbindungen Acrylat/C₁₀-C₃₀-Alkylacrylat, Xanthangummi, Tapiokastärke enthält.

Die erfindungsgemäße Zubereitung kann erfindungsgemäß vorteilhaft ein oder mehrere konservierend wirkende Stoffe enthalten. Erfindungsgemäß bevorzugte Ausführungsformen sind dann dadurch gekennzeichnet, dass die Zubereitung Methylparaben, Propylparaben, und/oder Phenoxyethanol enthält.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO₁,₅ und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Erfindungsgemäß besonders bevorzugte Ausführungsformen der vorliegenden Erfindung sind dadurch gekennzeichnet, dass die Zubereitung eine oder mehrere lipophile Bestandteile gewählt aus der Gruppe der Verbindungen Myristylmyristat, Octyldodecanol, C₁₂-C₁₅-Alkylbenzoat, Butylenglycoldicaprylat/Dicaprat, Ceatylalkohol enthält.

Es ist erfindungsgemäß bevorzugt, wenn die erfindungsgemäße Zubereitung Tocopherylacetat und/oder Tocopherol enthält.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin und/oder β-Alanin sowie Licochalcon A.

Vorteilhaft im Sinne der vorliegenden Erfindung sind Zubereitungen zur Pflege der Haut. Sie können dem kosmetischen Lichtschutz, der Hautpflege (beispielsweise zur Prophylaxe und Behandlung der Hautalterung) und dekorativen Kosmetik dienen.

Die Zubereitungen können sprühbar oder auf einem Träger (z.B. Tuch) dargereicht werden.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

### Vergleichsversuch

Der erfinderische Effekt konnte mit dem folgenden Vergleichsversuch belegt werden:
Es wurden die folgenden Zubereitungen hergestellt:

| | **1** | **2** |
|---|---|---|
| **INCI-Name(n)** | **m [%]** | **m [%]** |
| Alcohol Denat. | 4,00 | 4,00 |
| Natrium Hydroxide | 0,60 | 0,75 |
| Cetearyl Alkohol + PEG-40 Castor Oil + Natrium Cetearyl Sulfat | 2,50 | 2,50 |
| Glyceryl Stearat SE | 0,65 | 0,65 |
| EDTA | 1,00 | 1,00 |
| Methylparaben | 0,30 | 0,30 |
| Phenoxyethanol | 0,20 | 0,20 |
| Bis-Ethylhexyloxyphenol Methoxyphenyl Triazin | 2,00 | 2,00 |
| Butyl Methoxydibenzoylmethan | 4,50 | 4,50 |
| **Octocrylene** | **5,00** | **5,00** |
| Phenylbenzimidazol Sulfonsäure | 1,00 | 1,00 |
| Glycerin | 5,00 | 5,00 |
| C12-15 Alkyl Benzoat | 6,50 | 6,50 |
| Myristyl Myristat | 1,00 | 1,00 |
| Octyldodecanol | 5,50 | 5,50 |
| Parfum | 0,40 | 0,40 |
| Titandioxid | 2,00 | 2,00 |
| **Glycyrrhetinsäure** | **0,10** | |
| Cetyl Alkohol | 2,00 | 2,00 |
| Tapioca Stärke | 1,00 | 1,00 |
| Xanthan Gummi | 0,40 | 0,40 |
| Tocopherol Acetate | 0,50 | 0,50 |
| Wasser | Ad 100,0 | Ad 100,0 |

Die Zubereitungen wurden 4 Monate bei 40 °C gelagert und anschließend der Gehalt an dem UV-Filter Octocrylen mittels HPLC bestimmt. Für die Bestimmung des Gehaltes wurden zur Eichung der Messung Standard-Zubereitungen mit bekannter Konzentration verwendet.

**Ergebnis:**

| | Sollgehalt (Messung bevor die Zubereitung eingelagert wurde) % m/m | Rezeptur **2** ohne Glycyrrethinsäure % m/m nach 4 Monaten Lagerzeit | Rezeptur **1** mit Glycyrrethinsäure % m/m nach 4 Monaten Lagerzeit |
|---|---|---|---|
| Octocrylen | 5,0 | 4,6 | 4,9 |

**Fazit:** Durch Zusatz von Glycyrrethinsäure wird während der Lagerung deutlich weniger Octocrylen abgebaut.

### Beispiele

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

| | **1** | **2** | **3** | **4** | **5** | **6** | **7** | **8** | **9** | **10** | **11** |
|---|---|---|---|---|---|---|---|---|---|---|---|
| Acrylat/C10-30 Alkyl Acrylat Crosspolymer | | 0,2 | | 0,2 | | | | | 0,2 | | |
| Carbomer | | | | 0,2 | | | | | | | |
| Xanthan Gummi | 0,2 | 0,3 | | | | | | | | | 0,3 |
| Copolymer aus Vinylpyrrolidon und Acrylsäure | 0,4 | | 0,4 | | | | | | | | 0,2 |
| Tapioca Stärke | 1 | 1 | | 2 | 1,5 | | | 2 | | | |
| Distarch Phosphate | | | 1 | | | | 2 | | | | |
| Acrylonitrilemethacrylonitrile-methylmethacrylate Copolymer + Isopentane + Magnesium Hydroxide | | | | | | 0,3 | | | 0,5 | | |
| Carrageenan | | 0,2 | | | | | | | 0,25 | | |
| VP/Hexadecene Copolymer | | 1 | | 0,5 | 0,5 | 0,5 | 1 | | | | 1 |
| Acrylates/C12-22 Alkylmethacrylate Copolymer | | | 1,5 | | | | | 1 | 2 | | |
| C18-36 Acid Triglyceride | | | 1 | | | 1 | | 0,5 | | 1 | 0,5 |
| Natrium Stearoylglutamat | | 0,1 | 0,2 | 0,2 | | | | | | | |
| Sucrosepolystearat | | 0,8 | 1 | | | | | | | | |
| Cetearyl Alkohol + PEG-40 Castor Oil + Natrium Cetearyl Sulfat | 2,5 | | | | | 2 | | 2,5 | 2,25 | 2,5 | |
| Glyceryl Stearat SE | 0,7 | | | | 1 | 1 | | 1 | 0,75 | 1 | |
| Cetearyl Alcohol 90 + Sodium Cetearyl Sulfate 10 | | | | | 1,5 | | | | | | |
| Cetyl PEG/PPG-10/1 Dimethicone | | | | | | | 1 | | | | |
| Glyceryl Stearate Citrate | | | | | | | | | | | 2,5 |
| Isodecyl Neopentanoat | | 3 | | | | 2 | | | | 2 | |
| Myristyl Myristat | 1 | 1 | | | | 0,5 | | 0,5 | | | 0,5 |
| Butylenglycol Dicaprylat/Dicaprat | 1,5 | | 3 | | | | 3 | | 3 | | |
| C12-15 Alkyl Benzoat | 5 | | | 2 | 3 | 2 | 2 | | | | 2 |
| Caprylic/Capric Triglycerid | | | | 2 | | | | | | 3 | |
| Cetearylalkohol | | | | 1 | 1,5 | 0,5 | | 0,5 | | | |
| Cetyl Alkohol | 2 | | | | 2 | | | 1 | | | |
| Cyclomethicon | | | 5 | | | | | | | 1 | |
| Dicaprylylcarbonat | | 3 | | | 3 | | | | 2 | | 1 |
| Dimethicon | | | | | | 1 | 5 | | | | |
| Octyldodekanol | 4 | 5,5 | 3 | | 3,5 | | 4 | 3,5 | 6 | 2 | 4 |
| Hexyldecyl Laurate | | | | | | 3 | | | | 1 | |
| Isopropyl Palmitate | 1 | | | | | | 2 | | | 2 | 2 |
| Bis-Ethylhexyloxyphenol Methoxyphenyl Triazin | 2 | 1 | 1,5 | 2,5 | | | | 1 | 2 | 2 | 1 |
| Butyl Methoxydibenzoylmethan | 4,5 | 3 | 2 | | 4,5 | 4 | 4 | 3 | 4 | | 4,5 |
| Diethylamino Hydroxybenzoyl Hexyl Benzoat | | | 2 | 3 | | | | 1 | | 5 | |
| Ethylhexyl Methoxycinnamat | | | | | | | | | 0,5 | 5 | |
| Ethylhexyltriazone | | | | 1 | 0,5 | | | | | | |
| Homosalat | | | | 5 | | | 2 | | | | |
| Octocrylen | 5 | 4 | 8 | 5 | 5,5 | 6 | 4 | 7 | 6 | 3 | 5 |
| Octylsalicylat | | | | 3 | | 4 | 2 | 1 | | | 1 |
| Polysilicon-15 | | 2 | | | | 3 | | | | | 2 |
| Phenylbenzimidazol Sulfonsäure | 1 | 2 | | | | 2 | 2 | | | | |
| Titandioxid | 2 | | 4 | 2 | 1,5 | | | | 2 | | |
| Tris-(biphenyl)-1,3,5 triazin | | | 2 | | | | | | 1 | | |
| Tocopherol Acetate | 0,5 | 1 | 0,5 | 0,5 | 1 | 0,5 | 1 | 0,5 | 1 | 0,5 | 0,5 |
| Glycerin | 5 | 5 | 5 | 3 | 6 | 4 | 7 | 5 | 3 | 5 | 4 |
| Glycyrrhetinsäure | 0,1 | 0,1 | 0,2 | 0,1 | 0,15 | 0,1 | 0,1 | 0,1 | 0,1 | 0,1 | 0,1 |
| Alcohol Denat. | 5 | | 4 | 3 | 5 | 4 | 3 | 4 | 6 | 4,5 | 6 |
| Parfum | 0,4 | 0,4 | 0,3 | 0,3 | 0,4 | 0,3 | 0,5 | 0,4 | 0,3 | 0,4 | 0,4 |
| EDTA | 1 | 1 | 1 | 1 | 1 | 1 | 1 | 1 | 1 | 1 | 1 |
| Farbstoffe, Neutralisationsmittel, Konservierungsmittel etc | q.s. | q.s. | q.s. | q.s. | q.s. | q.s | q.s | q.s | q.s | q.s | q.s |
| Wasser | ad 100 | ad 100 | ad 100 | ad 100 | ad 100 | ad 100 | ad 100 | ad 100 | ad 100 | ad 100 | ad 100 |

## Patentansprüche

1. Kosmetische Zubereitung enthaltend
a) Glycyrrhetinsäure,
b) 2-Ethylhexyl-2-cyano-3,3-diphenylacrylat (Octocrylen) und
c) einen oder mehrere Parfümstoffe, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 2-Phenylbenzimidazol-5-sulfonsäuresalze und/oder Titandioxid als weitere UV-Filter enthält.

2. Verwendung von Glycyrrhetinsäure zur Stabilisierung von 2-Ethylhexyl-2-cyano-3,3-diphenylacrylat (Octocrylen) in kosmetischen Zubereitungen.

3. Kosmetische Zubereitung nach Anspruch 1 oder Verwendung nach Anspruch 2, **dadurch gekennzeichnet, dass** die Zubereitung einen oder mehrere weitere UV-Filter gewählt aus der Gruppe der Verbindungen Phenylen-1,4-bis-(2-benzimidazyl)-3,3'-5,5'-tetrasuifonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethyl-silyl)oxy]disiloxanyl]propyl]-phenol; 3-(4-Methylbenzyliden)campher; 3-Benzylidencampher; Ethylhexylsalicylat; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert,-Butyi)-4'-methoxydibenzoylmethan; Homomenthylsalicylat; 2-Ethylhexyl-2-hydroxybenzoat;; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoäsäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

4. Verwendung nach einem der vorhergehenden Ansprüche, **dadurch gekennzeichnet, dass** die Zubereitung einen oder mehrere Parfümstoffe enthält.

5. Kosmetische Zubereitung oder Verwendung nach einem der vorhergehenden Ansprüche, **dadurch gekennzeichnet, dass** die Zubereitung einen oder mehrere Parfümstoffe gewählt aus der Gruppe der Verbindungen Linalool, Benzyl Benzoate, Hydroxyisohexyl 3-Cyclohexene Carboxaldehyd, Hexyl Cinnamal, Benzyl Salicylate, Citronellol, Coumarin, enthält.

6. Kosmetische Zubereitung oder Verwendung nach einem der vorhergehenden Ansprüche, **dadurch gekennzeichnet, dass** die Zubereitung Glycyrrhetinsäure in einer Konzentration von 0,01 bis 1 Gew.-%, bezogen auf das Gesamtgewicht der Zubereitung, enthält.

7. Kosmetische Zubereitung oder Verwendung nach einem der vorhergehenden Ansprüche, **dadurch gekennzeichnet, dass** die Zubereitung 2-Ethylhexyl-2-cyano-3,3-diphenylacrylat (Octocrylen) in einer Konzentration von 0,5 bis 7,5 Gew.-%, bezogen auf das Gesamtgewicht der Zubereitung, enthält.

8. Kosmetische Zubereitung oder Verwendung nach einem der vorhergehenden Ansprüche, **dadurch gekennzeichnet, dass** die Zubereitung in Form einer Emulsion vorliegt.

9. Kosmetische Zubereitung nach einem der vorhergehenden Ansprüche, **dadurch gekennzeichnet, dass** die Zubereitung in Form einer O/W-Emulsion vorliegt.

10. Kosmetische Zubereitung oder Verwendung nach einem der vorhergehenden Ansprüche, **dadurch gekennzeichnet, dass** die Zubereitung 4-(tert.-Butyl)-4'-methoxydibenzoylmethan als weiteren UV-Filter enthält.

11. Verwendung nach einem der vorhergehenden Ansprüche, **dadurch gekennzeichnet, dass** die Zubereitung 2,4-Bis-{[4-(2-ethyl-hexytoxy)-2-hydroxy]-phenyl}-6-(4-methoxyphenyl)-1,3,5-triazin (INCI: Bis-Ethylhexyloxyphenol Methoxyphenyl Triazin), 2-Phenylbenzimidazol-5-sulfonsäuresalze und/oder Titandioxid als weitere UV-Filter enthält.

12. Kosmetische Zubereitung oder Verwendung nach einem der vorhergehenden Ansprüche, **dadurch gekennzeichnet, dass** die Zubereitung als Verdickungsmittel eine oder mehrere Verbindungen gewählt aus der Gruppe der Verbindungen Acrylat/C-₁₀-C₃₀-Alkylacrylat, Xanthangummi, Tapiokastärke enthält.

13. Kosmetische Zubereitung oder Verwendung nach einem der vorhergehenden Ansprüche, **dadurch gekennzeichnet, dass** die Zubereitung Methylparaben, Propylparaben, und/oder Phenoxyethanol enthält.

14. Kosmetische Zubereitung oder Verwendung nach einem der vorhergehenden Ansprüche, **dadurch gekennzeichnet, dass** die Zubereitung eine oder mehrere lipophile Bestandteile gewählt aus der Gruppe der Verbindungen Myristylmyristat, Octyldodecanol, C₁₂-C₁₅-Alkylbenzoat, Butylenglycoldicaprylat/Dicaprat, Ceatylalkohol enthält.

15. Kosmetische Zubereitung oder Verwendung nach einem der vorhergehenden Ansprüche, **dadurch gekennzeichnet, dass** die Zubereitung Tocopherylacetat und/oder Tocopherol enthält.

## Claims

1. Cosmetic preparation comprising
a) glycyrrhetinic acid,
b) 2-ethylhexyl 2-cyano-3,3-diphenylacrylate (octocrylene) and
c) one or more perfumes, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), 2-phenylbenzimidazole-5-sulfonic acid salts and/or titanium dioxide as further UV filters.

2. Use of glycyrrhetinic acid for stabilizing 2-ethylhexyl 2-cyano-3,3-diphenylacrylate (octocrylene) in cosmetic preparations.

3. Cosmetic preparation according to Claim 1 or use according to Claim 2, **characterized in that** the preparation comprises one or more further UV filters selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; hexyl 2-(4'-diethylamino-2'-hydroxybenzoyl)benzoate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane/dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tris(biphenyl)-1,3,5-triazine; 2,4-bis(4'-dineopentylaminobenzalmalonate)-6-(4"-butylaminobenzoate)-s-triazine, 4-dicyanomethylene-2,6-dimethyl-1,4-dihydropyridine-N-(ethyloxysulfate ester salt), titanium dioxide, zinc oxide, merocyanine, piperazine derivatives.

4. Use according to any of the preceding claims, **characterized in that** the preparation comprises one or more perfumes.

5. Cosmetic preparation or use according to any of the preceding claims, **characterized in that** the preparation comprises one or more perfumes selected from the group of compounds comprising linalool, benzyl benzoate, hydroxyisohexyl 3-cyclohexene carboxaldehyde, hexyl cinnamal, benzyl salicylate, citronellol, coumarin.

6. Cosmetic preparation or use according to any of the preceding claims, **characterized in that** the preparation comprises glycyrrhetinic acid at a concentration of 0.01 to 1% by weight, based on the total weight of the preparation.

7. Cosmetic preparation or use according to any of the preceding claims, **characterized in that** the preparation comprises 2-ethylhexyl 2-cyano-3,3-diphenylacrylate (octocrylene) at a concentration of 0.5 to 7.5% by weight, based on the total weight of the preparation.

8. Cosmetic preparation or use according to any of the preceding claims, **characterized in that** the preparation is in the form of an emulsion.

9. Cosmetic preparation according to any of the preceding claims, **characterized in that** the preparation is in the form of an O/W emulsion.

10. Cosmetic preparation or use according to any of the preceding claims, **characterized in that** the preparation comprises 4-(tert-butyl)-4'-methoxydibenzoylmethane as further UV filter.

11. Use according to any of the preceding claims, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), 2-phenylbenzimidazole-5-sulfonic acid salts and/or titanium dioxide as further UV filters.

12. Cosmetic preparation or use according to any of the preceding claims, **characterized in that** the preparation comprises as thickeners one or more compounds selected from the group of compounds comprising acrylate/C₁₀-C₃₀-alkyl acrylate, xanthan gum, tapioca starch.

13. Cosmetic preparation or use according to any of the preceding claims, **characterized in that** the preparation comprises methylparaben, propylparaben and/or phenoxyethanol.

14. Cosmetic preparation or use according to any of the preceding claims, **characterized in that** the preparation comprises one or more lipophilic constituents selected from the group of compounds comprising myristyl myristate, octyldodecanol, C₁₂-C₁₅-alkyl benzoate, butylene glycol dicaprylate/dicaprate, cetyl alcohol.

15. Cosmetic preparation or use according to any of the preceding claims, **characterized in that** the preparation comprises tocopheryl acetate and/or tocopherol.

## Revendications

1. Préparation cosmétique, contenant :
a) de l'acide glycyrrhizique,
b) de l'acrylate de 2-éthylhexyl-2-cyano-3,3-diphényle (octocrylène) et
c) un ou plusieurs parfums, **caractérisée en ce que** la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin), des sels de l'acide 2-phénylbenzimidazole-5-sulfoniqueet/ou du dioxyde de titane en tant que filtres UV supplémentaires.

2. Utilisation d'acide glycyrrhizique pour la stabilisation d'acrylate de 2-éthylhexyl-2-cyano-3,3-diphényle (octocrylène) dans des préparations cosmétiques.

3. Préparation cosmétique selon la revendication 1 ou utilisation selon la revendication 2, **caractérisée en ce que** la préparation contient un ou plusieurs filtres UV supplémentaires choisis dans le groupe de composés constitué par les sels de l'acide phénylène-1,4-bis-(2-benzimidazyl)-3,3'-5,5'-tétrasulfonique ; les sels de l'acide 2-phénylbenzimidazole-5-sulfonique ; le 1,4 di(2-oxo-10-sulfo-3-bornylidène-méthyl)-benzène et ses sels ; les sels de l'acide 4-(2-oxo-3-bornylidène-méthyl)benzène-sulfonique ; les sels de l'acide 2-méthyl-5-(2-oxo-3-bornylidène-méthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétramethyl-1-[(triméthylsilyl)oxy]disiloxanyl]propyl]-phénol ; le 3-(4-méthylbenzylidène)camphre ; le 3-benzylidène-camphre ; le salicylate d'éthylhexyle ; l'ester hexylique de l'acide 2-(4'-diéthylamino-2'-hydroxybenzoyl)-benzoïque ; l'acide téréphtalidène-dicamphre-sulfonique ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; l'ester 2-éthylhexylique de l'acide 4-méthoxycinnamique ; l'ester isoamylique de l'acide 4-méthoxycinnamique ; la 2-hydroxy-4-méthoxybenzophénone ; la 2-hydroxy-4-méthoxy-4'-méthylbenzophénone ; la 2,2'-dihydroxy-4-méthoxybenzophénone ; le 4-(tert.-butyl)-4'-méthoxydibenzoylméthane ; le salicylate d'homomenthyle ; le 2-hydroxy-benzoate de 2-éthylhexyle ; le benzalmalonate de diméthicodiéthyle ; le copolymère de 3-(4-(2,2-bis-éthoxycarbonylvinyl)-phénoxy)-propényl)-méthoxysiloxane/diméthylsiloxane ; la dioctylbutylamidotriazone (INCI : Diethylhexyl-Butamidotriazone) ; la 2,4-bis-[5-1-(diméthyl-propyl)benzoxazol-2-yl-(4-phényl)-imino]-6-(2-éthylhexyl)-imino-1,3,5-triazine de n° CAS 288254-16-0) ; l'ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; la 2,4-bis-{[4-(2-éthyl-hexyloxy)-2-hydroxy]-phényl}-6-{4-méthoxyphényl)-1,3,5-triazine (INCI : Bis-Ethylhexyloxyphenol Methoxyphenyl Triazin) ; la 2,4,6-tris-(biphényl)-1,3,5-triazine ; la 2,4-bis-(4'-dinéopentylaminobenzalmalonate)-6-(4"-butylamino-benzoate)-s-triazine ; le sel de N-(éthyloxysulfate-ester) de 4-dicyanométhylène-2,6-diméthyl-1,4-dihydropyridine ; le dioxyde de titane, l'oxyde de zinc, la mérocyanine, les dérivés de pipérazine.

4. Utilisation selon l'une quelconque des revendications précédentes, **caractérisée en ce que** la préparation contient un ou plusieurs parfums.

5. Préparation cosmétique ou utilisation selon l'une quelconque des revendications précédentes, **caractérisée en ce que** la préparation contient un ou plusieurs parfums, choisis dans le groupe de composés constitué par le linalool, le benzoate de benzyle, l'hydroxyisohexyl-3-cyclohexène-carboxaldéhyde, l'hexyl-cinnamal, le salicylate de benzyle, le citronellol, la coumarine.

6. Préparation cosmétique ou utilisation selon l'une quelconque des revendications précédentes, **caractérisée en ce que** la préparation contient de l'acide glycyrrhizique en une concentration de 0,01 à 1 % en poids, par rapport au poids total de la préparation.

7. Préparation cosmétique ou utilisation selon l'une quelconque des revendications précédentes, **caractérisée en ce que** la préparation contient de l'acrylate de 2-éthylhexyl-2-cyano-3,3-diphényle (octocrylène) en une concentration de 0,5 à 7,5 % en poids, par rapport au poids total de la préparation.

8. Préparation cosmétique ou utilisation selon l'une quelconque des revendications précédentes, **caractérisée en ce que** la préparation se présente sous la forme d'une émulsion.

9. Préparation cosmétique selon l'une quelconque des revendications précédentes, **caractérisée en ce que** la préparation se présente sous la forme d'une émulsion H/E.

10. Préparation cosmétique ou utilisation selon l'une quelconque des revendications précédentes, **caractérisée en ce que** la préparation contient du 4-(tert.-butyl)-4'-méthoxy-dibenzoylméthane en tant que filtre UV supplémentaire.

11. Utilisation selon l'une quelconque des revendications précédentes, **caractérisée en ce que** la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin), des sels de l'acide 2-phénylbenzimidazole-5-sulfonique et/ou du dioxyde de titane en tant que filtres UV supplémentaires.

12. Préparation cosmétique ou utilisation selon l'une quelconque des revendications précédentes, **caractérisée en ce que** la préparation contient en tant qu'épaississant un ou plusieurs composés choisis dans le groupe de composés constitué par l'acrylate/acrylate d'alkyle en C₁₀-C₃₀, la gomme xanthane, l'amidon de tapioca.

13. Préparation cosmétique ou utilisation selon l'une quelconque des revendications précédentes, **caractérisée en ce que** la préparation contient du méthylparabène, du propylparabène et/ou du phénoxyéthanol.

14. Préparation cosmétique ou utilisation selon l'une quelconque des revendications précédentes, **caractérisée en ce que** la préparation contient un ou plusieurs constituants lipophiles choisis dans le groupe de composés constitué par le myristate de myristyle, l'octyldodécanol, le benzoate d'alkyle en C₁₂-C₁₅, le dicaprylate/dicaprate de butylène glycol, l'alcool c cétylique.

15. Préparation cosmétique ou utilisation selon l'une quelconque des revendications précédentes, **caractérisée en ce que** la préparation contient de l'acétate de tocophéryle et/ou du tocophérol.
